# EUROPÄISCHE PATENTSCHRIFT

(11) **EP 2 228 433 B1**
(45) Veröffentlichungstag und Bekanntmachung des Hinweises auf die Patenterteilung: **05.04.2017**
(21) Anmeldenummer: 10001697.1
(22) Anmeldetag: 19.02.2010
(51) Int. Cl.: C12M 1/22

(54) **Zellkulturschale**
Cell culture dish
Boîte de culture cellulaire

(30) Priorität: 09.03.2009 DE 102009013673
(43) Veröffentlichungstag der Anmeldung: 15.09.2010
(73) Patentinhaber: EPPENDORF AG, 22339 Hamburg (DE)
(72) Erfinder: Gülzow, Nico, 22337 Hamburg (DE); Reimann, Thomas, 21502 Geesthacht (DE); Beese, Jochen, 22848 Norderstedt (DE); Kowanz, Ute, 22083 Hamburg (DE); Radacz, Yvonne, 73527 Schwäbisch Gmünd (DE)
(74) Vertreter: Hauck Patentanwaltspartnerschaft mbB

(56) Entgegenhaltungen:
- DE-A1-102007 027 273
- GB-A- 2 106 083
- JP-A- 2005 312 317
- US-A- 3 158 553

## Beschreibung

Die Erfindung bezieht sich auf eine Zellkulturschale.

Zellkulturschalen sind Schalen mit Deckel, die im biologischen oder medizinischen Labor zur Zellkultur und zur Kultivierung von Mikroorganismen (Mikroben und Pilze) und höheren Zellen (z.B. Säugerzellen, Zellgewebe etc) genutzt werden. In runder, durchsichtiger Ausführung werden sie auch als "Petrischale" bezeichnet.

Die US 3 055 808 offenbart eine Petrischale insbesondere zur Anzucht von Mikroorganismen umfassend eine flache zylindrische Schale und einen Deckel. Die Schale hat einen oberen, in Umfangsrichtung sich erstreckenden und den Deckel abdichtenden seitlichen Rand. Der tassenförmige Deckel hat die gleiche Form aber einen größeren Durchmesser, sodass er lose auf die Schale passt. Der Deckel hat in einer Ecke zwischen einer Deckwand und einer umlaufenden Seitenwand einen druckempfindlichen, permanent klebenden Klebstoff zum lösbaren Befestigen des Deckels an der Schale und hermetischen Abdichten der Verbindung zwischen Deckel und Schale. Der Klebstoff erstreckt sich in Umfangsrichtung entlang des oberen äußeren Randes der Schale und entlang der inneren Oberflächen des Umfanges der Deckwand an der Ecke. Der Deckel besteht aus einem flexiblen Material, das so ausgeführt ist, dass er sich bei Ausübung eines seitlichen Druckes auf seine Seitenwand verbiegt und sich infolgedessen die Dichtung zwischen dem Deckel und der Schale löst, wobei der Klebstoff bei Entfernung des Deckels von der Schale an dem Deckel verbleibt.

Die Petrischale dient der Kultivierung von Mikroorganismen, insbesondere von Bakterien. Bei aufgesetztem Deckel ist die Petrischale hermetisch geschlossen. Der Deckel hat keine Belüftungsstellung. Beim Auftrag des Klebstoffes kann es zu einer erhöhten Verkeimung der Petrischale kommen, sodass der Aufwand für die Sterilisation erhöht wird. Die Klebeeigenschaft des Klebstoffes kann durch lange Lagerungszeit, erhöhte Temperatur im Brutschrank, Einwirkung tiefer Temperaturen oder Kontakt mit Kulturmedium verändert werden. Hierdurch kann die Abdichtung zwischen Deckel und Schale beeinträchtigt werden. Auch können Inhaltsstoffe des Klebstoffs das Kulturmedium kontaminieren.

Die Firma BD Biosciences, San José, USA bietet unter der Bezeichnung "BD Falcon" und der Artikelnummer 35106 Petrischalen mit einem Durchmesser von 50 und einer Höhe von 9 mm an. Die Petrischalen bestehen aus Polystyrol und haben einen Deckel, der einen abdichtenden Sitz auf der Schale hat. Hierfür weist die Schale am oberen Rand einer umlaufenden Seitenwand einen konischen Dichtsitz und der Deckel an der Innenwand einer Deckelseitenwand einen komplementären Dichtsitz auf. Der Deckel ist mit seinem Dichtsitz klemmend auf den Dichtsitz der Schale aufsetzbar, d.h. sobald der Deckel aufgesetzt ist; liegt eine reibschlüssige Verbindung vor. Dabei geht die Länge der Seitenwand des Deckels nicht über den konischen Bereich der Seitenwand der Schale hinaus. Daraus resultiert der komplementäre Dichtsitz, aber beim Öffnen kann keine Hebelwirkung den Deckel verformen. Die Petrischale hat keine definierte Belüftungsstellung mit einem Belüftungsspalt zwischen Schale und Deckel. Diese Petrischale ist, sobald der Deckel einmal abdichtend aufgesetzt ist, nicht einfach zu öffnen und es muss dafür ein vermehrter Kraftaufwand eingebracht werden. Eine Einhandhandhabung dieser Petrischale, insbesondere beim Öffnen ist nicht möglich. Der Anwender muss, um die reibschlüssige Verbindung zu überwinden, die Petrischale mit einer Hand an der unteren Schale fassen und festhalten und mit der anderen Hand den Deckel der Petrischale kraftvoll hochziehen. Bei einer solchen Handhabung ist es unvermeidbar, dass es zu Turbulenzen im Kulturmedium in der Petrischale kommt, wodurch empfindliche Zellen, insbesondere manipulierte Zellen (z.B. transformierte und/oder infizierte Zellen etc.), negativ beeinflusst werden, sich beispielsweise von dem Schalenboden lösen, und damit unbrauchbar werden. Zusätzlich geht mit diesen Turbulenzen eine sprühnebelartige Tröpchenbildung/Aerosolbildung einher, so dass bei Öffnung der Petrischale ein Sprühnebel/Aerolsol an Kulturmedium in die Umgebungsluft gerissen wird und diese kontaminiert. Dies ist insbesondere bei der Kultivierung von pathogenen Organismen in Zellen (Viren, Mycoplasma, Prionen etc.), oder von Zellen, die ein Toxin in das Kulturmedium sekretieren gefährlich und somit unerwünscht, da so der Anwender einem Gesundheitsrisiko ausgesetzt wird.

Zum Inkubieren bzw. Kultivieren werden Zellen bzw. Mikroorganismen in eine Zellkulturschale eingeführt. Bei einer verbreiteten Vorgehensweise wird die Schale durch Auflegen eines loses aufliegenden Deckels abgedeckt, um die Zellen bzw. Mikroorganismen zu schützen. Die Zellkulturschale wird in einen Brutschrank eingesetzt, in dem optimale Bedingungen für die Inkubation bzw. Kultivierung geschaffen werden, indem eine hierfür geeignete Atmosphäre und Temperatur eingestellt wird. Die Atmosphäre im Brutschrank wird in der Regel durch Luft mit einem bestimmten CO₂- und O₂- Gehalt unter einer bestimmten Luftfeuchtigkeit gebildet. Der lose aufliegende Deckel ermöglicht es, dass die im Brutschrank herrschende Atmosphäre auch innerhalb der Zellkulturschale, d.h. an den Zellen bzw. Mikroorganismen vorliegt. Ferner sind Zellkulturschalen bekannt, bei denen der Deckel durch punktuelle Noppen einen größeren Abstand von der Schale aufweist, sodass der Gasaustausch mit dem Inneren der Zellkulturschale stärker ausgeprägt ist.

Aus der US 5 520 302 ist eine rechteckige Petrischale zum Kultivieren von Mikroorganismen bekannt. Sie besitzt eine rechteckige Schale mit einer Bodenwand und einer umlaufenden Seitenwand, die von der Bodenwand emporsteht. Ferner weist die Zellkulturschale einen rechteckigen Deckel auf, der in zwei verschiedenen Positionen anbringbar ist. Der Deckel hat eine Deckwand und eine umlaufende äußere sowie eine innere Deckelseitenwand, die sich von der Deckwand aus nach unten erstrecken und eine endlose Nut begrenzen. Die Kontur der endlosen Nut entspricht dem oberen Ende der Seitenwand der Schale. Wenn der Deckel in einer ersten Position die Schale vollständig überdeckt, ist das obere Ende der Seitenwand daran gehindert, vollständig in die Nut einzugreifen, sodass kein Gasdurchgang zwischen dem Deckel und der Schale möglich ist. Wenn der Deckel um 180° gedreht in einer zweiten Position vollständig oberhalb der Schale positioniert wird, greift das obere Ende der Seitenwand in die Nut ein. Hierdurch wird ein Gasdurchgang zwischen Deckel und Schale verhindert.

Bei der bekannten Petrischale muss der Deckel in der korrekten Ausrichtung auf die Schale aufgesetzt werden, damit der Gasdurchgang zwischen Deckel und Schale ermöglicht wird. Zum gasdichten Verschließen der Petrischale muss der Deckel abgenommen und nach Drehung um 180° erneut aufgesetzt werden. Hierdurch ist die Handhabung der Petrischale erschwert. Außerdem können die Mikroorganismen bei diesen Manipulationen verunreinigt werden. Ferner kann sich die Atmosphäre im Inneren der Petrischale beim Umsetzen des Deckels an die Zusammensetzung der Umgebung anpassen, sodass die Kulturbedingungen im Inneren der Petrischale nicht erhalten bleiben.

Die US 4 675 298 offenbart eine Petrischale mit einer kreisrunden unteren Schale und einem darauf lose angeordneten Deckel. Vorsprünge sind am Umfang der Innenseite des Deckels angeordnet und darauf abgestimmte Vertiefungen sind auf dem Rand der Schale gebildet. Dies ermöglicht das Einstellen eines Zwischenraumes zwischen dem Deckel und der Schale für die Steuerung des Gasaustausches zwischen dem Inneren der Petrischale und der Atmosphäre. Zum Einstellen des Zwischenraumes muss der Deckel bezüglich der Schale verdreht werden, was den Einsatz beider Hände erfordert. Hierfür muss der Anwender die Petrischale gegebenenfalls zunächst einem Brutschrank entnehmen. Da der Deckel lose auf der Schale aufliegt, ist die Petrischale nicht flüssigkeitsdicht, auch wenn der Zwischenraum zwischen Schale und Deckel vollständig geschlossen ist. Infolgedessen kann es bei Manipulation der Petrischale zu einem Verschütten von Kulturmedium bzw. Mikroorganismen kommen.

Aus JP 2005-312317 A ist eine Zellkulturschale bekannt geworden, bei der in der Belüftungsposition der Deckel mit nach unten gerichteten Vorsprüngen an sechs Stellen des Innenumfangs des Deckels auf einem umlaufenden wulstförmigen Rand an der Außenseite der Seitenwand der Schale aufsitzt. In der Abdichtposition untergreift der rückspringende Bereich oberhalb eines an der Innenseite der Deckelseitenwand umlaufenden Vorsprungs, der Vorsprünge trägt, den Wulst am oberen Rand der Seitenwand der Schale. Das Herunterdrücken des Deckels aus der Belüftungsposition in die Abdichtposition erfordert Kräfte, da hierzu der obere Randbereich der Seitenwand umlaufend nach innen gedrückt bzw. die Deckelseitenwand umlaufend nach außen aufgeweitet werden muss. Das Lösen des Deckels von der Schale erfordert ebenfalls einen hohen Kraftaufwand und wird durch einen seitlichen vom Deckel vorstehenden Hebel erleichtert.

Davon ausgehend liegt der Erfindung die Aufgabe zugrunde, eine anwendungsfreundlichere Zellkulturschale zu schaffen. Unter anwendungsfreundlich ist insbesondere zu verstehen: einfache manuelle und/oder automatische Handhabung (wiederholbares Öffnen, wiederholbares Schließen, und/oder Tragen der Zellkulturschale) lediglich mit einer Hand bzw. einem Automatenwerkzeug. Des Weiteren kommt dem Begriff anwendungsfreundlich hier ebenfalls die Bedeutung risikoarm bezüglich potentieller Kontamination des Anwenders zu, d.h. das Risiko des Anwenders wird minimiert, kontaminiertem (infiziert oder toxisch) Kulturmedium ausgesetzt zu sein.

Die Aufgabe wird durch eine Zellkulturschale mit den Merkmalen des Anspruches 1 gelöst. Vorteilhafte Ausgestaltungen der Zellkulturschale sind in den Unteransprüchen angegeben.

Die erfindungsgemäße Zellkulturschale umfasst
- eine Schale mit einer Bodenwand und einer von dieser hochstehenden, umlaufenden Seitenwand,
- einen Deckel mit einer von einer Deckwand nach unten vorstehenden Deckelseitenwand, der in einer Abdichtposition abdichtend auf der Seitenwand aufsitzt, wobei die Deckelseitenwand die Seitenwand der Schale übergreift
- wobei die Kontaktflächen von Deckel und Seitenwand als Dichtsitz ausgebildet sind und Reibschlussmittel sind, die den Deckel in Abdichtposition an der Schale lösbar halten,
- wobei die Seitenwand der Schale mindestens eine seitlich vorstehende Federzunge aufweist, und die Deckelseitenwand auf die Federzunge in Belüftungsposition, in der zwischen Seitenwand und Deckel ein Belüftungsspalt vorhanden ist, aufsetzbar ist,
- wobei die Abdichtposition durch Andrücken des Deckels gegen die Schale ermöglicht wird und das Ablösen des Deckels von der Schale durch leichtes Zusammendrücken des Deckels an zwei diametral einander gegenüberliegenden Seiten mit einer einzigen Hand ausführbar ist.

Bei einer alternativen Lösung der erfindungsgemäßen Aufgabe nach Patentanspruch 2 weist der Deckel mindestens eine Federzunge auf, die durch vom unteren Rand der Deckelseitenwand ausgehende, parallele Schlitze der Deckelseitenwand begrenzt ist, die in einer Deckelseitenwand angeordnet und senkrecht zu deren Umfang auslenkbar ist, und an ihrem Innenumfang an einem rampenartigen oder keilförmigen Vorsprung auf dem Umfang der Seitenwand der Schale anliegt. Die übrigen Merkmale dieser Lösung entsprechen denen des Patentanspruchs 1.

Bei der erfindungsgemäßen Zellkulturschale kann die Schale nach dem Einbringen von Zellen oder Mikroorganismen durch Aufsetzen des Deckels abgedeckt werden. Der Deckel kann zunächst in Belüftungsposition angeordnet werden, in der der Belüftungsspalt das Innere der Zellkulturschale mit der Umgebung verbindet. Dabei kann der Deckel in beliebigen Positionen auf die Schale aufgesetzt werden. Der Anwender benötigt hierfür nur eine einzige Hand. In dieser Konfiguration ist die Zellkulturschale in einen Brutschrank einsetzbar, sodass das Innere der Zellkulturschale über den Belüftungsspalt mit der Atmosphäre im Brutschrank verbunden ist und diese an den Zellen oder Mikroorganismen in der Schale anliegt. Nach der Inkubation oder Kultivierung, z.B. wenn die Zellkulturschale aus dem Brutschrank entfernt werden soll, kann die Zellkulturschale durch Zusammendrücken von Deckel und Schale dicht geschlossen werden. Hierdurch wird die Abdichtposition erreicht, in der der Deckel abdichtend auf der Seitenwand aufsitzt. Dieses abdichtende Aufsitzen gewährleistet, dass beim Transport hochschwappende Kulturflüssigkeit nicht aus der Zellkulturschale austritt. Ferner gewährleistet das abdichtende Aufsitzen, dass die im Brutschrank eingestellte Atmosphäre in der Zellkulturschale länger erhalten bleibt oder sich langsamer ändert, als wenn der Deckel in Belüftungsposition angeordnet ist.

Die Erfindung bezieht mögliche Ausgestaltungen ein, die erhöhten Dichtigkeitsanforderungen genügen. Diese können z. B. flüssigkeitsdicht in dem Sinne sein, dass keine Flüssigkeit austritt, wenn in der Zellkulturschale eine Säule aus der Kulturflüssigkeit an dem Dichtbereich zwischen Deckel und Seitenwand ansteht. Ferner sind im Rahmen der Erfindung Ausgestaltungen möglich, bei denen der Deckel die Schale gasdicht abschließt. Dieses gasdichte Abschließen kann bei einer einfacheren Ausführung gegeben sein, wenn innerhalb und außerhalb der Zellkulturschale der gleiche Gasdruck herrscht. Bei einer weiter erhöhten Anforderungen entsprechenden Ausführung ist Gasdichtigkeit auch dann gegeben, wenn innerhalb und außerhalb der Zellkulturschale unterschiedliche Drucke herrschen (z. B. um 0,2 bar oder 0,5 bar verschiedene Drucke). Gasdichtigkeit kann auch dadurch erreicht werden, dass der Anwender gezielt einen Flüssigkeitsfilm in den Dichtbereich zwischen Schale und Deckel einbringt. Dies kann beispielsweise dadurch geschehen, dass der Anwender einen Teil eines in die Zellkulturschale eingebrachten Kulturmediums in den Dichtbereich "einschwappt". Dies kann der Anwender durch eine leichte Taumelbewegung bzw. Schwenkbewegung der Zellkulturschale erreichen.

Zum dichten Abschließen der Zellkulturschale braucht der Deckel nur gegen die auf einem Untergrund angeordnete oder in der Hand gehaltene Schale leicht gedrückt zu werden. Hierdurch wird der Belüftungsspalt geschlossen und sitzt der Deckel abdichtend auf der Seitenwand auf. Ein vorheriges Umsetzen des Deckels ist nicht erforderlich. Der Anwender kann somit die Zellkulturschale durch bloßes Herunterdrücken des Deckels auf die auf einem Untergrund angeordnete Schale schließen. Auch hierfür benötigt er nur eine einzige Hand, bzw, nur einen Finger. Infolgedessen kann er die Zellkulturschale leicht noch innerhalb eines Brutschrankes schließen. So wird zum einen vermieden, dass beim Transport Flüssigkeit austreten kann. Und zum anderen wir vermieden, dass sich die Atmosphäre im Inneren der Zellkulturschale an die außerhalb des Brutschranks vorherrschende Atmosphäre anpasst, d.h die ursprüngliche Brutschrankatmosphäre bleibt innerhalb der Zellkulturschale erhalten, obwohl diese sich nicht mehr im Brutschrank befindet. Insbesondere kann ein CO₂-Verlust vermieden werden, so dass ein konstanter pH-Wert in der Zellkulturschale über längere Zeit aufrecht erhalten bleiben kann. Insbesondere ist dies gesichert, wenn der Anwender gezielt einen Flüssigkeitsfilm in den Dichtbereich einbringt. Das Risiko der Kontamination von Zellen bzw. Mikroorganismen beim Anordnen des Deckels in Belüftungsposition und in Abdichtposition ist vermindert. In Abdichtposition kann ein Überschwappen bzw. Auslaufen von Kulturmedium aus der Zellkulturschale vermieden werden. Somit ist ebenfalls das Risiko des Anwenders vermindert, kontaminierten Medium ausgesetzt zu sein. Insgesamt hat die erfindungsgemäße Zellkulturschale günstigere Anwendungseigenschaften als der eingangs erwähnte Stand der Technik.

Der Deckel ist in Abdichtposition bzw. in Belüftungsposition lösbar an der Schale angeordnet sein, um den Deckel von der Schale abnehmen zu können und auf die Zellen bzw. Mikroorganismen in der Schale zuzugreifen. Die Grundfläche der erfindungsgemäßen Zellkulturschale kann verschieden ausgestaltet sein, insbesondere rechteckig, oval oder kreisrund. Bevorzugt ist sie kreisrund.

Die Erfindung bezieht Ausgestaltungen ein, bei denen der Belüftungsspalt ungleichmäßig ausgebildet ist. Beispielsweise kann der Belüftungsspalt zwischen Deckel und Seitenwand ausgehend von einem Aufsatzbereich, in dem der Deckel in Belüftungsposition auf der Seitenwand aufsitzt, mit zunehmendem Abstand von diesem Aufsitzbereich zunehmen.

Bei der Erfindung ist die Federzunge elastisch. Der Deckel kann unter Überwindung elastischer Rückstellkräfte aus der Belüftungsposition - hier liegt der Deckel mit seiner Deckelseitenwand lose auf dem Auflageelement auf- in die Abdichtposition gebracht werden, und auch wieder aus der Abdichtposition zurück in die Belüftungsposition. Bei Abnahme des Deckels kehrt die Federzunge in ihre Ausgangslage zurück, sodass der Deckel erneut lose auf der Federzunge abgelegt werden kann. Diese Ausbildung ermöglicht ein wiederholtes Öffnen, Belüften und abdichtendes Verschließen der Zellkulturschale, wie es bei sich über mehrere Tage erstreckenden zelltechnischen Untersuchungen üblich ist. Diese Ausbildung eignet sich somit insbesondere für Zellkulturschalen, die nach einer Inkubation bzw. Kultivierung aus dem Brutschrank entnommen werden, um die Zellkultur oder Mikroorganismen unter dem Mikroskop zu untersuchen oder ein Medium zuzugeben, und die danach erneut zur weiteren Inkubation bzw. Kultivierung in den Brutschrank eingesetzt werden, wobei diese Behandlung gegebenenfalls mehrfach wiederholt werden kann. Vor der Entnahme aus dem Brutschrank wird der Deckel in Abdichtposition gebracht um die CO₂ angereicherte Atmosphäre innerhalb der Zellkulturschale zu erhalten und ein Überschwappen und Austreten des Kulturmediums während des Transport zu verhindern; und vor dem Zurückstellen in den Brutschrank zwecks weiterer Inkubation wird der Deckel in Belüftungsposition gebracht. Gemäß dieser Ausgestaltung kann die Zellkulturschale als Einwegartikel ausgeführt sein, insbesondere aus Kunststoff.

Gemäß einer Ausgestaltung weist die Schale einen nach außen von der Seitenwand vorstehenden Greifrand auf. Der Greifrand ermöglicht insbesondere die einfache manuellen und/oder Automatenhandhabung der Zellkulturschale. Der Greifrand kann sich über einen oder mehrere Umfangsabschnitte der Seitenwand der Schale erstrecken. Auch kann er sich ununterbrochen oder über den gesamten Umfang der Seitenwand erstrecken. Der Greifrand erleichtert das Halten bzw. Transportieren der Schale. Gemäß einer Ausgestaltung ist das nachgiebige Auflageelement ein Abschnitt des Greifrandes der Schale. Die Federzunge kann von einem Abschnitt des Greifrandes gebildet sein. Die Federzunge kann im unbelasteten Zustand spitzwinklig zur Bodenwand ausgerichtet sein, sodass sie bei Verlagerung des Deckels von der Belüftungsposition in die Abdichtposition näher zur Bodenwand geschwenkt wird. Die Federzunge kann an einem Ende fest mit der Seitenwand verbunden sein, erstreckt sich parallel zur Bodenwand und weist am anderen Ende einen zum Deckel hin vorstehenden Vorsprung auf, auf den die Deckelseitenwand aufsetzbar ist. Bei dieser Ausgestaltung kann die Federzunge Teil eines ringförmig umlaufenden Greifrandes der Schale sein. Ein Schwenken der Federzunge näher zur Bodenwand hin ist möglich, ohne dass die unbelastete Federzunge spitzwinklig zur Bodenwand gerichtet ist.

Der Vorsprung der Federzunge kann genutzt werden, um den Deckel in spitzwinkliger Ausrichtung zur Schale am unteren Rand der Deckelseitenwand in einem Spalt zwischen dem Vorsprung und der Seitenwand der Schale einzuklemmen. In dieser Schrägstellung des Deckels ist zwischen Deckel und Schale ein Belüftungsspalt vorhanden, dessen Breite ausgehend von dem Vorsprung in Umfangsrichtung der Schale bis zu einer dem Vorsprung gegenüber liegenden Stelle zunimmt, und zwar auf beiden Seiten des Vorsprungs. Diese weitere Belüftungsposition gewährleistet gegenüber der Belüftungsposition, in der der Deckel durch die Mittel zum Halten gehalten wird, einen verstärkten Gasaustausch mit der Umgebung. Dies kann erwünscht sein, wenn die mit Zellen besiedelte Zellkulturschale nach einem längeren Zeitraum außerhalb des Brutschranks in den Brutschrank zurückgestellt wird, um dort eine schnelle Akklimatisierung an die dort vorherrschende Atmosphäre zu gewährleisten. In der weiteren Belüftungsposition ist die Schale weiterhin abgedeckt und vor Verunreinigungen geschützt. Der Deckel wird weiterhin sicher von der Schale gehalten und hat auch in dieser Position nur Kontakt mit der Schale, d.h. er berührt beispielsweise nicht den Brutschrank selber. Somit ist auch in dieser Position das Kontaminationsrisiko minimiert. Zum sicheren Halten des Deckels in der weiteren Belüftungsposition kann die Deckelseitenwand außen mit einem radial nach außen vorstehenden, umlaufenden Randflansch versehen sein und/oder der Vorsprung einen radial nach innen vorstehenden Klemmvorsprung haben.

Die Federzunge kann durch vom unteren Rand der Deckelseitenwand ausgehende, parallele Schlitze der Deckelseitenwand begrenzt sein. Nach Anspruch 2 liegt die Federzunge an einem rampenartigen oder keilförmigen Vorsprung auf dem Umfang der Seitenwand der Schale an. Infolgedessen gleitet die Federzunge beim Andrücken des Deckels gegen die Schale über die Rampe und wird zunehmend ausgelenkt.

Der Deckel und die Schale haben Mittel zum Abdichten haben, die gewährleisten, dass der Deckel in Abdichtposition abdichtend auf der Seitenwand aufsitzt. Hierfür sind die Kontaktflächen von Deckel und Seitenwand als Dichtsitze ausgebildet sein. Dabei können die Kontaktflächen besonders passgenau ausgeführt sein. Gemäß einer bevorzugten Ausgestaltung weisen die Mittel zum Abdichten einen Hinterschnitt auf, der auf der Außenseite der Seitenwand der Schale in Umfangsrichtung der Seitenwand verläuft, und einen nach innen vorspringenden Dichtbereich, der auf der Innenseite der Deckelseitenwand in Umfangsrichtung der Seitenwand verläuft, wobei der vorspringende Dichtbereich in Abdichtposition den Hinterschnitt hintergreift und in Belüftungsposition außer Eingriff mit dem Hinterschnitt ist. In der Abdichtposition wird somit eine formschlüssige Verbindung eingegangen.

Gemäß einer bevorzugten Ausgestaltung liegt ein konischer Abschnitt am Innenumfang der umlaufenden Deckelseitenwand abdichtend am Mantel der Seitenwand der Schale an und/oder liegt ein konischer Abschnitt am Mantel der Seitenwand abdichtend am Innenumfang der umlaufenden Deckelseitenwand an. Im Bereich des Hinterschnittes und des vorspringenden Dichtbereichs der ersten Ausführung und des konischen Abschnittes der zweiten Ausführung wird eine besonders hohe Flächenpressung und dementsprechend ein verstärkter Dichteffekt erreicht.

Reibschlussmittel werden von Dichtsitzen an Deckel und Schale gebildet, die in Abdichtposition zusammenwirken. Rastmittel können vom Hinterschnitt und dem damit zusammenwirkenden vorspringenden Dichtbereich gebildet sein, wobei der vorspringende Dichtbereich hinter dem Hinterschnitt verrastet wird.

Die beschriebenen Mittel zum lösbaren Halten werden leicht durch Zusammendrücken des Deckels an zwei diametral einander gegenüber liegenden Seiten überwunden. Dieses Zusammendrücken hat eine Verformung des Deckels zur Folge (z.B. wird der ursprünglich kreisrunde Deckel kurzzeitig oval verformt), es setzt eine Hebelwirkung ein, die das Ablösen des Deckels von der Schale erleichtert. Ein solches Zusammendrücken ist ebenfalls mit einer einzigen Hand ausführbar, so dass der Anwender leicht den Deckel von der Zellkulturschale ablösen kann, ohne dabei an der Schale ziehen zu müssen. Sobald das Zusammendrücken beendet wird, nimmt der Deckel seine ursprüngliche Form wieder an. Durch diese Art der Ablösung wird vermieden, dass in der mit Zellkulturmedium befüllten und mit Zellen besiedelten Zellkulturschale Turbulenzen im Zellkulturmedium entstehen, die zum einen nachträglich für die Zellen sein können und zum anderen zur sprühnebelartige Tröpchenbildung/Aerosolbildung des Zellkulturmediums führen, dem der Anwender ausgesetzt werden würde und der zur gesundheitlichen Beeinträchtigung dieses führen könnte.

Die erfindungsgemäße Zellkulturschale besteht im Wesentlichen aus Kunststoff. Geeignete Kunststoffe sind ausgewählt aus der Gruppe der transparenten Kunststoffe wie Polystyrol (PS), Polypropylen (PP), Polymethylpenten Polycarbonat (PC), Polymethylacrylat (PMMA), Polymethacrylmethylimid (PMMI) und Cyclo-Olefin-Copolymer (COC) sowie Gemischen und/oder Co-Polymerisaten aus wenigstens zwei dieser Kunststoffe. Gemäß einer Ausgestaltung besteht die Schale und/oder der Deckel aus mindestens einem Kunststoff. Für Schale und Deckel können dieselben oder verschiedene Kunststoffe zum Einsatz kommen. Ferner können Schale und/oder Deckel aus mehreren Kunststoffen bestehen. Beispielsweise kann der Deckel am Innenumfang der Deckelseitenwand einen eingelegten oder eingespritzten Dichtring aus einem elastischen Material haben, der mit einer am Außenumfang der Seitenwand der Schale umlaufenden Dichtfläche Mittel zum Abdichten bildet. Ein weiteres Beispiel für einen aus mehreren Kunstoffen bestehende Schale und/oder Deckel ist eine Zellkulturschale, die in wenigstens einem ausgewählten Bereich der Schale und/oder des Deckels einen besonders transparenten und/oder reflexarmen Kunststoff aufweist. So ist es möglich Bereiche auszubilden, die sich besonders für die optische Observation der Zellen eignen.

Bevorzugt bestehen Deckel und Kunststoff aus demselben Material. Ein bevorzugtes Material ist beispielsweise Polystyrol.

Weiterhin bevorzugt sind Schale und/oder Deckel zumindest bereichsweise aus einem hoch transparenten Kunststoff wie PMMA, PMMI und/oder COC. Bevorzugt sind Deckel und Schale im Bereich der Böden transparent. Weiterhin bevorzugt sind sie insgesamt transparent. Ein transparenter Kunststoff ermöglicht optische Untersuchungen von Kulturen, die in der Zellkulturschale angeordnet sind, ohne die Zellkulturschale zu öffnen. Bevorzugt ist hierfür die Zellkulturschale ganz oder teilweise aus einem glasklaren Kunststoff.

Die erfindungsgemäße Zellkulturschale kann weiterhin Oberflächenmodifikationen aufweisen, die die Anbindung und das Wachstum von Zellen unterstützen. Insbesondere sind solche Oberflächenmodifikationen, die durch Plasmapolymerisation aufgebracht werden bevorzugt.

Die Erfindung wird nachfolgend anhand der anliegenden Zeichnungen eines Ausführungsbeispieles näher erläutert. In den Zeichnungen zeigen:
- Fig. 1: eine Zellkulturschale in Belüftungsposition in einer teilweise geschnittenen Perspektivansicht;
- Fig. 2: dieselbe Zellkulturschale in Abdichtposition in einer teilweise geschnittenen Perspektivansicht;
- Fig. 3: dieselbe Zellkulturschale in Unteransicht;
- Fig. 4: die Schale derselben Zellkulturschale in einer teilweisen perspektivischen Seitenansicht;
- Fig. 5: dieselbe Zellkulturschale beim Öffnen des Deckels in einer Perspektivansicht schräg von der Seite;
- Fig. 6: dieselbe Zellkulturschale in Belüftungsposition in einem vergrößerten vertikalen Teilschnitt;
- Fig. 7: dieselbe Zellkulturschale in Abdichtposition in einem vergrößerten vertikalen Teilschnitt;
- Fig. 8: dieselbe Zellkulturschale in Abdichtposition mit abdichtendem Flüssigkeitskeil in einem vergrößerten vertikalen Teilschnitt;
- Fig. 9: der abdichtende Flüssigkeitskeil in einem weiter vergrößerten vertikalen Teilschnitt;
- Fig. 10: dieselbe Zellkulturschale in einer weiteren Belüftungsposition mit schräggestelltem Deckel in einer Perspektivansicht schräg von der Seite;
- Fig. 11: eine weitere Ausgestaltung der Zellkulturschale mit in der Deckelseitenwand integrierten Federzungen in einer vergrößerten Perspektivansicht schräg von oben und von der Seite.

In der vorliegenden Anmeldung beziehen sich die Angaben "oben" und "unten" auf eine Ausrichtung der Zellkulturschale, bei der die Schale mit ihrem Boden auf einer horizontalen Unterlage ruht.

Bei der Erörterung verschiedener Ausführungsbeispiele werden für entsprechende Merkmale dieselben Bezugsziffern verwendet und unterschiedliche Ausprägungen eines weiteren Ausführungsbeispieles durch einen hochgestellten Anstrich (') gekennzeichnet.

Die Zellkulturschale 1 umfasst eine Schale 2 und einen Deckel 3 mit jeweils kreisförmiger Grundfläche. Die Schale 2 hat eine Bodenwand 4, von der eine umlaufende Seitenwand 5 hochsteht.

Von der Außenseite der Seitenwand 5 steht etwa auf halber Höhe ein im Wesentlichen kreisringförmiger, umlaufender Greifrand 6 nach außen vor. Der Greifrand 6 umfasst vier Federzungen 7, die gleichmäßig über den Umfang der Schale 2 verteilt sind. Die Federzungen 7 sind an ihrem festen Ende einteilig mit dem Greifrand 6 verbunden und erstrecken sich im Wesentlichen in der Ebene des Greifrandes 6. An ihrem freien Ende haben sie einen nach oben von dem übrigen Greifrand 6 hochstehenden Vorsprung 8 bzw. Nocken.

Der Greifrand 6 ist in Bereichen, in denen die Federzungen 7 angeordnet sind, unterbrochen. Es ist auch möglich, den Greifrand 6 ununterbrochen auszuführen, wobei die Federzungen 7 durch im Greifrand 6 angeordnete Schlitze begrenzt sein können.

Der Deckel 3 weist eine Deckwand 9 auf. Vom Rand der Deckwand 9 steht eine umlaufende Deckelseitenwand 10 nach unten vor.

Am Innenumfang der Deckelseitenwand 10 ist ein konischer Abschnitt 11 vorhanden, der einen Dichtsitz des Deckels 3 bildet. Dem konischen Abschnitt 11 ist ein umlaufender konischer Abschnitt 12 am Außenumfang der Seitenwand 5 zugeordnet.

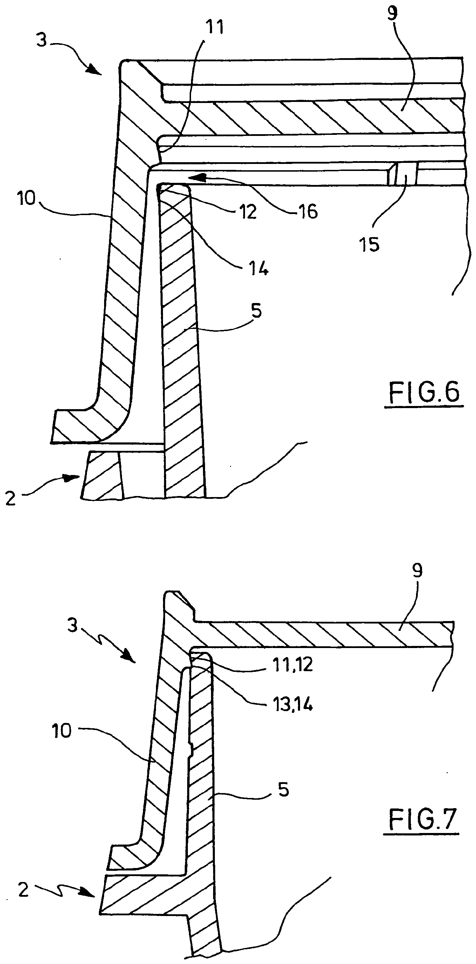

Gemäß Fig. 6 bis 8 ist der konische Abschnitt 11 auf einem auf dem Innenumfang der Deckelseitenwand 10 umlaufenden vorspringenden Dichtbereich 13 angeordnet. Der konische Abschnitt 12 ist an einem Hinterschnitt 14 am Außenumfang der Seitenwand 5 angeordnet.

Der konische Abschnitt 11 und der konische Abschnitt 12 erweitern sich jeweils zu Deckwand 9 hin.

Der Außendurchmesser am oberen Ende der Seitenwand 5 ist um etwa 1 bis 3 Zehntelmillimeter größer als der Innendurchmesser der Deckelseitenwand 10 am unteren Ende des vorspringenden Dichtbereiches 13. Bevorzugt beträgt die Differenz etwa 1,5 Zehntelmillimeter.

Der Hinterschnitt 14 ist etwa 1 bis 5 Hundertstelmillimeter tief. Bevorzugt ist er 3 Hundertstelmillimeter tief.

Der konische Abschnitt 11 und der zugeordnete konische Abschnitt 12 können beispielsweise eine Höhe von 0,3 mm - 1,5mm, vorzugsweise von 0,5 mm - 1 mm und besonders bevorzugt eine Höhe von 0,5 mm aufweisen.

Der Deckel 3 weist am Innenumfang der Deckelseitenwand 10 keilförmige Rippen 15 auf, die sich bis zum unteren Rand des vorspringenden Dichtbereiches 13 erstrecken. Dies ist nur in Fig. 6 gezeigt. Die Rippen 15 sind keilförmig, wobei ihre Höhe nach oben zum vorspringenden Dichtbereich 13 zunimmt. Das untere Ende der Rippen 15 ist in einem Abstand vom vorspringenden Dichtbereich 13 angeordnet, der beispielsweise einen Drittel oder der Hälfte der Höhe der Deckelseitenwand 10 entspricht. Die Rippen 15 zentrieren den Deckel 3 auf der Schale 2 und erleichtern so das Verrasten.

Mehrere Rippen 15 sind gleichmäßig über den Innenumfang der Deckelseitenwand 10 verteilt. Bevorzugt sind mindestens drei Rippen 15 vorhanden. Im Beispiel sind es sechs Rippen 15, wobei jedoch nur eine gezeigt ist.

Schale 2 und Deckel 3 können so ausgeführt sein, dass sie in den äußeren und inneren Abmessungen im Wesentlichen mit bekannten Zellkulturschalen übereinstimmen. Insbesondere kann der Innendurchmesser der Seitenwand 5 im Bereich von 30 bis 150 mm angesiedelt sein, beispielsweise bei 55 mm. Die Höhe der Seitenwand 5 kann im Bereich von 5 bis 20 mm angesiedelt sein; sie kann beispielsweise 15 mm betragen.

Schale 2 und Deckel 3 sind beispielsweise aus Polystyrol hergestellt.

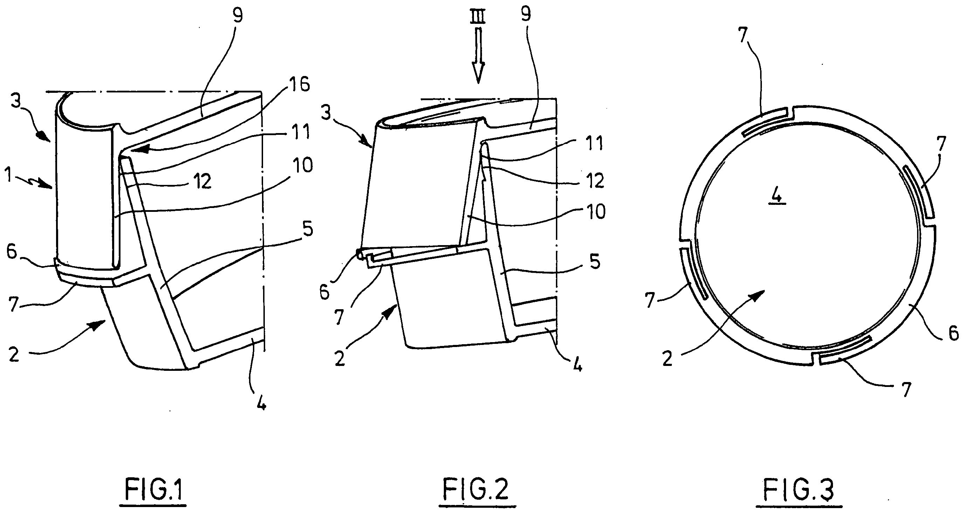

Wenn gemäß Fig. 1 und 6 der Deckel 3 lose auf die Schale 2 aufgesetzt ist, sodass der untere Rand der Deckelseitenwand 10 auf den Vorsprüngen 8 aufliegt, ist ein Belüftungsspalt 16 zwischen Schale 2 und Deckel 3 vorhanden. Die Höhe des Belüftungsspaltes 16 ist in einem Bereich von 0,1 mm - 1 mm, vorzugsweise 0,3 - 0,7 mm und besonders bevorzugt bei 0,5 mm angesiedelt. Das Aufsetzen des Deckels 3 auf die Schale 2 in Belüftungsposition kann leicht von einer einzigen Hand durchgeführt werden.

Gemäß Fig. 2 und 7 kann der Deckel 3 durch Drücken gegen die Schale 2 in Abdichtposition gebracht werden. Hierbei lenkt der untere Rand der Deckelseitenwand 10 die Federzungen 7 etwas nach unten aus. Die Seitenwand 5 gleitet mit ihrem oberen Rand über die Rippen 15 und wird hierdurch zentriert. Zugleich wird die Seitenwand 5 etwas radial zusammengedrückt und die Deckelseitenwand 10 etwas radial aufgeweitet. Schließlich schnappt der vorspringende Dichtbereich 13, unter dem Hinterschnitt 14 und die konischen Dichtbereiche 11, 12 liegen abdichtend aneinander an. Infolgedessen wird die Schale 2 durch den aufgedrückten Deckel 3 dicht verschlossen. In dieser Position wird der Deckel 3 an der Schale 2 durch das formschlüssige Ineinandergreifen von Hinterschnitt 14 und vorspringendem Dichtbereich 13 fixiert. Die Abdichtung wird durch die Klemmkraft zwischen den Abschnitten 11, 12 bewirkt. In der Abdichtposition liegen somit sowohl eine formschlüssige wie auch eine reibschlüssige Verbindung vor. Durch diese Kombination liegt ein sicherer, insbesondere flüssigkeitsdichter Verschluss vor, der sich aber auch wieder einfach - durch Zusammendrücken der Deckelseitenwand 10 (hier stellen sich Hebelkräfte ein) sich bilden- lösen lässt.

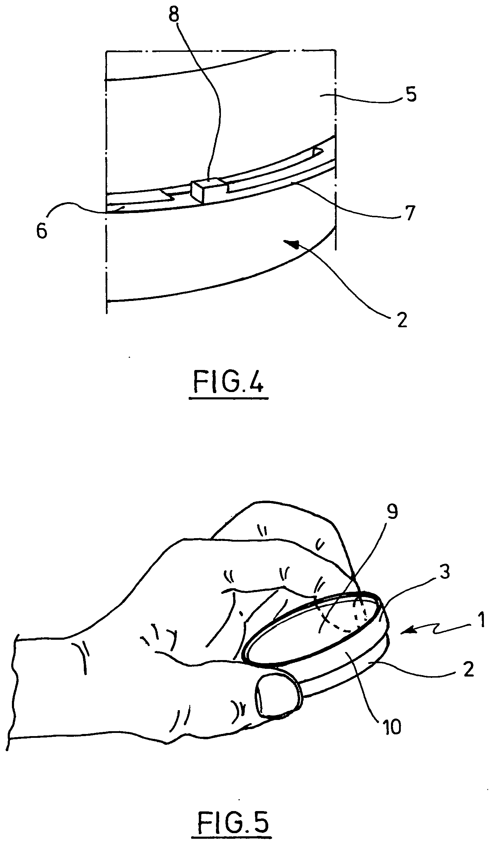

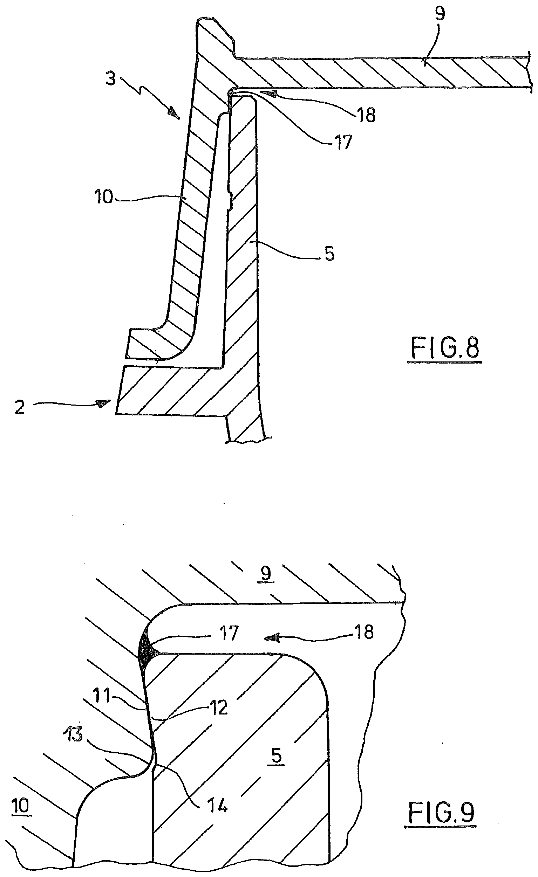

Zur zusätzlichen Abdichtung kann gemäß Fig. 8 und 9 ein Flüssigkeitskeil 17 zwischen den oberen Rand der Seitenwand 5 und das obere Ende der Deckelseitenwand 10 eingebracht werden. Hierfür ist ein kleiner umlaufender Restspalt 18 zwischen der Seitenwand 5 und der Deckwand 9 vorhanden. Durch den Flüssigkeitskeil 18 wird die Abdichtung zwischen Schale 2 und Deckel 3 verbessert. Gemäß Fig. 5 wird die Zellkulturschale geöffnet, indem mittels Zeigefinger und Daumen seitlich gegen den unteren, kreisförmigen Rand der Deckelseitenwand 10 gedrückt wird, sodass sich dieser zu einer Ellipse verformt. Das hat zur Folge, dass sich die ebene Deckwand 9 und der konische Abschnitt 11 der Deckelseitenwand 10 verformen. In dem um 90° zur Kraftrichtung der Finger gelegenen Bereich wird in Folge dessen der Eingriff des Vorsprunges 13 in den Hinterschnitt 14 aufgehoben und der Deckel 3 löst sich von der Schale 2 ab.

Die erfindungsgemäße Zellkulturschale 1 hat bessere Anwendungseigen-schaften als herkömmliche Zellkulturschalen. Insbesondere können Schale 2 und Deckel 3 leicht miteinander verbunden werden. Das Auf und Abnehmen des Deckels 3 ist mit nur einer einzigen Hand möglich. Die Zellkulturschale ist in Abdichtposition abdichtend geschlossen, sodass der CO₂-Gehalt im Inneren der Zellkulturschale 1 nahezu konstant gehalten werden kann. Ein Überschwappen durch einen Belüftungsspalt, der bei einem nur aufgelegten Deckel vorhanden ist, kann nicht mehr erfolgen, wenn der Deckel die Schale abdichtend verschließt. Die Belüftungsposition und die Abdichtposition können ohne Einfluss auf die jeweils andere Position vollwertig genutzt werden.

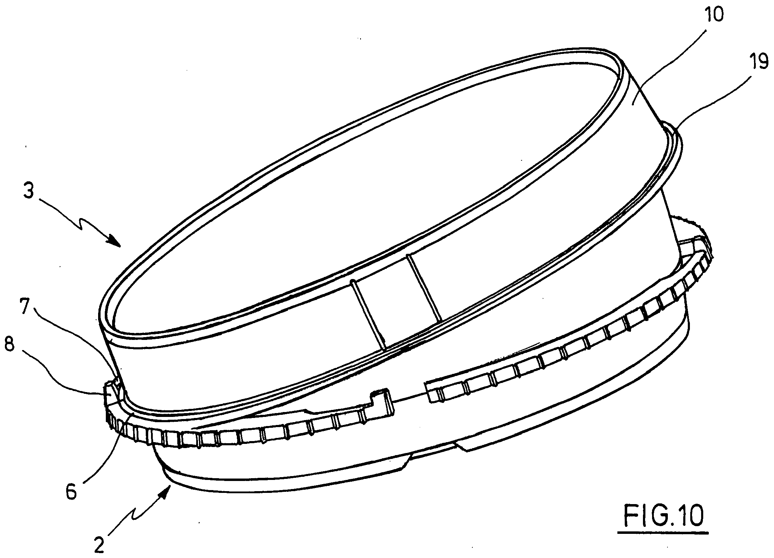

Gemäß Fig. 10 kann der Deckel 3 in einer weiteren Belüftungsposition an der Schale 2 gehalten werden, in der der Deckel 3 spitzwinklig zur Schale 2 ausgerichtet ist. Hierfür wird ein nach außen vorstehender, umlaufender Randflansch 19 des Deckels 2 an der Innenseite des Vorsprunges 8 einer Federzunge 7 angelegt. Der Randflansch 19 ist in dem Spalt zwischen dem Vorsprung 8 und der Seitenwand 5 eingeklemmt, wodurch der Deckel 3 in der gezeigten Ausrichtung fixiert ist. In dieser weiteren Position wird ein stark sich erweiternder Belüftungsspalt erreicht, der einen besonders guten Gasaustausch ermöglicht. Dennoch ist die Schale 2 oben abgedeckt, wobei auch hier der Deckel 3 lediglich in Kontakt ist mit der Schale 2. Insbesondere berührt der Deckel 3 nicht den Untergrund, der kontaminiert sein kann, und so wird vermieden, dass Kontaminationen während der Belüftung in die Schale gelangen.

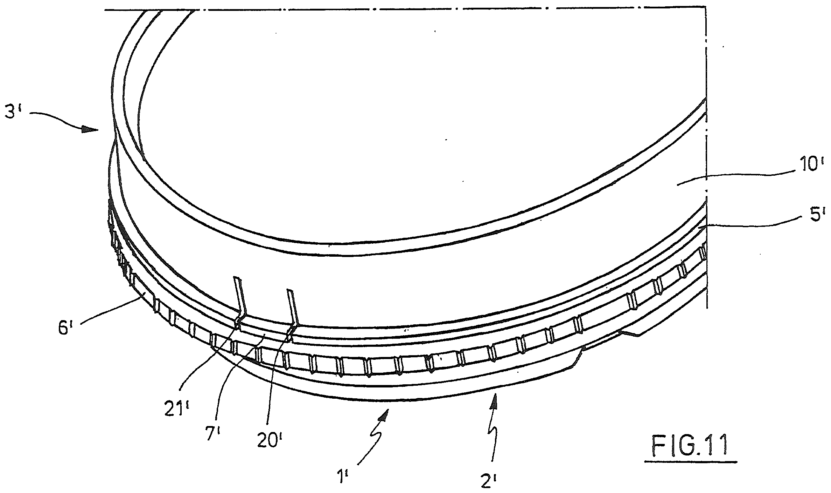

Die Zellkulturschalel' gemäß Fig. 11 unterscheidet sich von der Vorbeschriebenen dadurch, dass der Rand 6 ununterbrochen umläuft und keine Federzungen 7 aufweist. Stattdessen sind Federzungen 7' in der Deckelseitenwand 10' vorhanden. Hierzu weist die Deckelseitenwand 10' von ihrem unteren Rand ausgehende, parallele Schlitze 20', 21' auf, die die Federzungen 7' begrenzen. Im Beispiel sind über den Umfang der Deckelseitenwand 10' drei Federzungen 7' verteilt, wobei nur eine gezeigt ist.

Die Federzungen 7'haben - nicht gezeigt - nach Innen stehende, vorspringende Führungsnocken. Die Führungsnocken der Federzungen 7' sind auf - nicht gezeigten - keilförmig oder wulstartig seitlich vorspringenden Führungsflächen auf dem unteren Teil der Seitenwand 5' geführt. Die Führungsflächen können beliebig laufen, z.B. könne die ganz umlaufen oder auch unterbrochen umlaufen. Bei lose aufgesetzte Deckel 3' sind die Federzungen 7' an den nicht gezeigten Keilflächen in erhöhten Position gegenüber der Schale 2' abgestützt. Beim Zusammendrücken von Deckel 3' und Schale 2' gleiten die Federzungen 7' auf den Führungsflächen und werden nach außen abgelenkt. Schließlich schnappt der vorspringende Dichtbereich 13' hinter den Hinterschnitt 14' und der Deckel 3' ist in Abdichtposition abdichtend an der Schale 2' fixiert.

## Patentansprüche

1. Zellkulturschale umfassend
- eine Schale (2) mit einer Bodenwand (4) und einer von dieser hochstehenden, umlaufenden Seitenwand (5),
- einen Deckel (3) mit einer von einer Deckwand (9) nach unten vorstehenden Deckelseitenwand (10), der in einer Abdichtposition abdichtend auf der Seitenwand (5) aufsitzt, wobei die Deckelseitenwand (10) die Seitenwand (5) der Schale (2) übergreift,
- wobei die Kontaktflächen von Deckel (3) und Seitenwand (5) als Dichtsitz (13, 14) ausgebildet sind und Reibschlussmittel sind, die den Deckel (3) in Abdichtposition an der Schale (2) lösbar halten,
- wobei die Seitenwand (5) der Schale (2) mindestens eine seitlich vorstehende Federzunge (7) aufweist und die Deckelseitenwand (10) auf die Federzunge (7) in Belüftungsposition, in der zwischen Seitenwand (5) und Deckel (3) ein Belüftungsspalt (16) vorhanden ist, aufsetzbar ist,
- wobei die Abdichtposition durch Andrücken des Deckels gegen die Schale ermöglicht wird und
- das Ablösen des Deckels (3) von der Schale (2) durch leichtes Zusammendrücken des Deckels (3) an zwei diametral einander gegenüberliegenden Seiten mit einer einzigen Hand ausführbar ist.

2. Zellkulturschale aufweisend:
- eine Schale (2) mit einer Bodenwand (4) und einer von dieser hochstehenden, umlaufenden Seitenwand (5)
- einen Deckel (3) mit einer von einer Deckwand (9) nach unten vorstehenden Deckelseitenwand (10), der in einer Abdichtposition abdichtend auf der Seitenwand (5) aufsitzt, wobei die Deckelseitenwand (10) die Seitenwand (5) der Schale (2) übergreift,
- wobei die Kontaktflächen von Deckel (3) und Seitenwand (5) als Dichtsitz (13, 14) ausgebildet sind,
- und mindestens eine Federzunge hat, die durch vom unteren Rand der Deckelseitenwand (10) ausgehende, parallele Schlitze der Deckelseitenwand (10) begrenzt sind, die in einer Deckelseitenwand (10) angeordnet ist und senkrecht zu deren Umfang auslenkbar ist, und an ihren Innenumfang an einem rampenartigen oder keilförmigen Vorsprung auf dem Umfang der Seitenwand der Schale anliegt,
- wobei die Abdichtposition durch Andrücken des Deckels gegen die Schale ermöglicht wird und
- das Ablösen des Deckels (3) von der Schale (2) durch leichtes Zusammendrücken des Deckels (3) an zwei diametral einander gegenüberliegenden Seiten mit einer einzigen Hand ausführbar ist.

3. Zellkulturschale nach Anspruch 1, bei der die Schale (2) mindestens drei Federzungen (7) aufweist.

4. Zellkulturschale nach Anspruch 1 oder 3, bei der die Schale (2) an einander gegenüberliegenden Abschnitten der Seitenwand (5) Federzungen (7) aufweist.

5. Zellkulturschale nach Anspruch 4, die ein erstes Paar Federzungen (7) an zwei einander gegenüber liegenden Abschnitten der Seitenwand (5) und ein zweites Paar Federzungen (7) an zwei weiteren einander gegenüberliegenden Abschnitten der Seitenwand (5) aufweisen, wobei das zweite Paar gegenüber dem ersten Paar um 90° versetzt angeordnet ist.

6. Zellkulturschale nach einem der Ansprüche 1 und 3 bis 5, bei der die Federzunge (7) an einem Ende fest mit der Seitenwand (5) verbunden ist, sich parallel zur Bodenwand (4) erstreckt und am anderen Ende einen zum Deckel (3) hin vorstehenden Vorsprung (8) aufweist, auf den die Deckelseitenwand (10) aufsetzbar ist.

7. Zellkulturschale nach einem der Ansprüche 1 bis 6, die einen außen von der Seitenwand (5) vorstehenden Greifrand (6) aufweist.

8. Zellkulturschale nach Anspruch 7, bei der die Federzungen (7) ein Abschnitt des Greifrandes (6) sind.

9. Zellkulturschale nach einem der Ansprüche 1 bis 8, bei der die Mittel zum Abdichten einen an der Außenseite der Seitenwand (5) umlaufenden Hinterschnitt (14) und einen auf der Innenseite der Deckelseitenwand (10) vorspringenden Dichtbereich (13) haben, wobei der vorspringende Dichtbereich (13) in Abdichtposition den Hinterschnitt (14) hintergreift und in Belüftungsposition außer Eingriff mit dem Hinterschnitt (14) ist.

10. Zellkulturschale nach einem der Ansprüche 1 bis 9, bei der in Abdichtposition ein konischer Abschnitt (11) am Innenumfang der umlaufenden Deckelseitenwand (10) abdichtend am Mantel der Seitenwand (5) der Schale (2) und/oder ein konischer Abschnitt (12) am Mantel der Seitenwand (5) abdichtend am Innenumfang der umlaufenden Deckelseitenwand (10) anliegt.

11. Zellkulturschale nach einem der Ansprüche 1 bis 10 mit einer Schale 3 aus Kunststoff und/oder einem Deckel (2) aus Kunststoff.

## Claims

1. A cell culture dish, comprising:
- a dish (2) with a bottom wall (4) and a circumferential side wall (5) standing upward from the same,
- a lid (3), having a lid side wall (10) projecting downwardly the lid top wall (9) which in a sealing position sits sealingly on the side wall (5), the lid side wall (10) is extending over the side walls of the dish (2),
- contact surfaces of lid (3) and side wall (5) being formed as sealing seat (13, 14) and defining frictionally fitting means, which resiliently retains a lid (3) on the dish (2) in the sealing position,
- the side wall (5) of dish (2) has at least one laterally projecting spring tongue (7) onto which a lid side wall (10) in the aeration position of lid (3) is settable, in the aeration position an aeration gap (16) being formed between side wall (5) and lid (3),
- the sealing position is achieved by pressing the lid against the dish and
- the detaching of lid (3) from dish (2) can be carried out by an easy compression of the lid (3) by a single or one-hand operation from opposing sides.

2. A cell culture dish, comprising
- a dish (2) with the bottom wall (4) and a circumferential side wall (5) standing upward from the same,
- a lid (3) having a side wall (10) projecting downwardly the lid top wall (9) which in a sealing position sits sealingly on the side wall (5), the lid side wall (10) is extending over the side wall of dish (2),
- the contact surfaces of lid (3) and side wall (5) being formed as sealing seat (13, 14),
- at least one side wall (5) of dish (2) having at least one laterally projecting spring tongue which is bordered by parallel slits of the side wall (10) which start from the lower edge of the side wall (10) and which are located in the side wall (10) and can be deflected orthogonal to the perimeter thereof, the spring tongue on the inner circumference engaging a ramp-like projection on the circumference of the side wall of the dish,
- whereby a sealing position is achieved by pressing the lid against the dish and
- the detachment of lid (3) from dish (2) is achieved by an easy compression of the lid from two diametrically opposing sides by a one-hand operation.

3. The cell culture dish of claim 1, wherein the dish (2) has at least three spring tongues (7).

4. The cell culture dish of claim 1 or 3, wherein dish (2) on opposing portions of side wall (5) has spring tongues (7).

5. The cell culture dish, wherein a first pair of spring tongues (7) are provided on two opposing portions of side wall (5) and a second pair of spring tongues (7) being provided on two further opposing portions of side wall (5), the second pair is arranged staggered relative to the first pair by about 90°.

6. The cell culture dish of one of claims 1 and 3 to 5, wherein the spring tongue (7) on one end is fixedly connected to side wall (5) and extends parallel to bottom wall (4) and comprising on the other end a projection (8) extending towards lid (3) onto which the side wall (10) of lid (3) can be placed.

7. The cell culture dish according to one of the claims 1 to 6, wherein side wall (5) has a gripping edge (6) that projects from the side wall (5) at the outer side.

8. The cell culture dish of claim 7, wherein the spring tongues (7) form a portion of the gripping edge (6).

9. The cell culture dish of one of the claims 1 to 8, wherein the means for sealing have an undercut (14) circulating on the outer side of side wall (5) and a sealing region (13) projecting on the inner side of lid side wall (10), the projecting sealing region (13) gripping behind the undercut (14) in the sealing position and being out of engagement with the undercut (14) in the aeration position.

10. The cell culture dish of one of claims 1 to 9, wherein in the sealing position a conical section (11) on the inside perimeter of the circumferential lid side wall (10) sits sealingly close to the curved surface of side wall (5) of the dish (2) and/or a conical section (12) on the curved surface of a side wall (5) sits sealingly close to the inside perimeter of the circumferential lid side wall (10).

11. The cell culture dish of one of the claims 1 to 10, wherein the dish (3) is made of plastic material with a dish (3) of plastics and/or a lid (2) of plastic material.

## Revendications

1. Boîte de culture cellulaire comprenant
- une boîte (2) avec une paroi inférieure (4) et une paroi latérale périphérique (5) élevée depuis celle-ci,
- un couvercle (3) avec une paroi latérale de couvercle (10) saillant vers le bas depuis une paroi de recouvrement (9) qui repose dans une position d'étanchéité de manière étanchéifiante sur la paroi latérale (5), dans laquelle la paroi latérale de couvercle (10) déborde sur la paroi latérale (5) de la boîte (2),
- dans laquelle les faces de contact du couvercle (3) et de la paroi latérale (5) sont réalisées en tant que siège d'étanchéité (13, 14) et sont des moyens d'entraînement par friction qui maintiennent de manière amovible le couvercle (3) dans la position d'étanchéité sur la boîte (2),
- dans laquelle la paroi latérale (5) de la boîte (2) présente au moins une languette à ressort (7) saillant latéralement et la paroi latérale de couvercle (10) peut être posée sur la languette à ressort (7) dans la position d'aération dans laquelle un interstice d'aération (16) est présent entre paroi latérale (5) et couvercle (3),
- dans laquelle la position d'étanchéité est permise par appui du couvercle contre la boîte et
- le détachement du couvercle (3) de la boîte (2) peut être réalisé par légère compression du couvercle (3) sur deux côtés diamétralement opposés l'un à l'autre avec une seule main.

2. Boîte de culture cellulaire comprenant :
- une boîte (2) avec une paroi inférieure (4) et une paroi latérale périphérique (5) élevée depuis celle-ci,
- un couvercle (3) avec une paroi latérale de couvercle (10) saillant vers le bas depuis une paroi de recouvrement (9) qui repose dans une position d'étanchéité de manière étanchéifiante sur la paroi latérale (5), dans laquelle la paroi latérale de couvercle (10) déborde sur la paroi latérale (5) de la boîte (2),
- dans laquelle les faces de contact du couvercle (3) et de la paroi latérale (5) sont réalisées en tant que siège d'étanchéité (13, 14),
- et a au moins une languette à ressort qui sont limitées par des fentes parallèles de la paroi latérale de couvercle (10) partant du bord inférieur de la paroi latérale de couvercle (10), laquelle est disposée dans une paroi latérale de couvercle (10) et peut être déviée perpendiculairement à sa périphérie, et repose sur sa périphérie interne au niveau d'une saillie de type rampe ou en forme de cale sur la périphérie de la paroi latérale de la boîte,
- dans laquelle la position d'étanchéité est permise par appui du couvercle contre la boîte et
- le détachement du couvercle (3) de la boîte (2) peut être réalisé par légère compression du couvercle (3) sur deux côtés diamétralement opposés l'un à l'autre avec une seule main.

3. Boîte de culture cellulaire selon la revendication 1, dans laquelle la boîte (2) présente au moins trois languettes à ressort (7).

4. Boîte de culture cellulaire selon la revendication 1 ou 3, dans laquelle la boîte (2) présente des languettes à ressort (7) sur des sections opposées l'une à l'autre de la paroi latérale (5).

5. Boîte de culture cellulaire selon la revendication 4, qui présente une première paire de languettes à ressort (7) sur deux sections opposées l'une à l'autre de la paroi latérale (5) et une seconde paire de languettes à ressort (7) sur deux autres sections opposées l'une à l'autre de la paroi latérale (5), dans laquelle la seconde paire est disposée de manière décalée de 90° par rapport à la première paire.

6. Boîte de culture cellulaire selon une des revendications 1 et 3 à 5, dans laquelle la languette à ressort (7) est reliée à une extrémité de manière fixe à la paroi latérale (5), s'étend parallèlement à la paroi inférieure (4) et présente à l'autre extrémité une saillie (8) saillant vers le couvercle (3) sur laquelle la paroi latérale de couvercle (10) peut être posée.

7. Boîte de culture cellulaire selon une des revendications 1 à 6, qui présente un bord de saisie (6) saillant à l'extérieur de la paroi latérale (5).

8. Boîte de culture cellulaire selon la revendication 7, dans laquelle les languettes à ressort (7) sont une section du bord de saisie (6).

9. Boîte de culture cellulaire selon une des revendications 1 à 8, dans laquelle les moyens pour l'étanchéification ont une contre-dépouille périphérique (14) sur le côté externe de la paroi latérale (5) et une région d'étanchéité (13) saillant sur le côté interne de la paroi latérale de couvercle (10), dans laquelle la région d'étanchéité saillante (13) s'engage avec la contre-dépouille (14) dans la position d'étanchéité et est hors d'engagement avec la contre-dépouille (14) dans la position d'aération.

10. Boîte de culture cellulaire selon une des revendications 1 à 9, dans laquelle une section conique (11) repose dans la position d'étanchéité sur la périphérie interne de la paroi latérale de couvercle périphérique (10) de manière étanchéifiante sur l'enveloppe de la paroi latérale (5) de la boîte (2) et/ou une section conique (12) repose sur l'enveloppe de la paroi latérale (5) de manière étanchéifiante sur la périphérie interne de la paroi latérale de couvercle périphérique (10).

11. Boîte de culture cellulaire selon une des revendications 1 à 10 avec une boîte 3 en plastique et/ou un couvercle (2) en plastique.
